# EUROPÄISCHE PATENTSCHRIFT

(11) **EP 2 095 830 B1**
(45) Veröffentlichungstag und Bekanntmachung des Hinweises auf die Patenterteilung: **18.05.2011**
(21) Anmeldenummer: 08000895.6
(22) Anmeldetag: 18.01.2008
(51) Int. Cl.: A61L 2/10, E05B 1/00

(54) **UV-Desinfektionsvorrichtung**
UV disinfection device
Dispositif de désinfection par rayonnement ultraviolet

(43) Veröffentlichungstag der Anmeldung: 02.09.2009
(73) Patentinhaber: Buchal, Thomas, 58093 Hagen (DE)
(72) Erfinder: Buchal, Thomas, 58093 Hagen (DE)
(74) Vertreter: Kötter, Ulrich

(56) Entgegenhaltungen:
- WO-A-2006/007729
- WO-A-2007/070512
- US-A- 3 314 746
- US-A1- 2002 146 343

## Beschreibung

Die Erfindung betrifft eine Vorrichtung zur bedarfsgerechten Desinfektion einer Armatur zum Öffnen einer Tür nach dem Patentanspruch 1.

Nach Angabe des Robert-Koch-Instituts befindet sich die Anzahl krankenhausbürtiger Infektionen, wie Wundinfektionen, Harnwegsinfektionen, Pneumonien oder Septiken auch in den entwickelten Staaten nach wie vor auf hohem Niveau. Sie wird entsprechend der Gesundheitsberichterstattung des Bundes für Deutschland derzeit mit ca. 500.000 bis 800.000 Fällen pro Jahr veranschlagt. Als Hauptursache gilt die für Kliniken und Pflegeheime charakteristische Häufung des Zusammentreffens pathorgener Keime mit imunsupprimierten Patienten auf engem Raum. Als bedeutsamster Übertragungsweg in Einrichtungen des Gesundheitswesens gilt die indirekte Kontamination. Hierbei gelangen Keime von infizierten oder lediglich kolonisierfien Personen durch Personen- oder Oberflächenkontakten zu potenziell gefährdeten Patienten. Insbesondere der Körperhygiene, hier besonders der Händedesinfektion, wird daher viel Aufmerksamkeit gewidmet. Ebenso stellt die intensive Reinigung / Desinfektion von Kontaktoberflächen, wie Türklinken, welche von vielen Personen stark frequentiert werden, eine bedeutsame Strategie dar.

Hygienemaßnahmen an häufig frequentierten Kontaktoberflächen müssen regelmäßig, können auf Grund des hohen personellen Aufwandes aber nicht beliebig häufig am Tage durchgeführt werden. Die Zwischenzeiten ermöglichen aber die Neukontamination und halten somit die Gefahr einer Übertragung von infektiösen Erregern aufrecht. An dieser Stelle sind Vorrichtungen erforderlich, welche eine selbstständige Reinigung der frequentierten Kontaktflächen ermöglichen.

Aus der US 3,314,746 A ist ein Türgriff bekannt, der aus Quarzglas ausgebildet ist und eine UV-Strahlungsquelle aufnimmt. Es ist ein abnehmbares Schild angeordnet, um die Sichtbarkeit der UV-Strahlung für den Benutzer zu minimieren. Eine weitere Anordnung zur Desinfektion eines Türgriffs mittels UV-Strahlung ist in der WO 2001/070512 A offenbart. Problematisch bei den vorbekannten Vorrichtungen ist jedoch, dass die Türarmaturen während des Desinfektionsvorgangs nicht nutzbar sind oder die Abschirmung selbst als Bedienfläche verwendet wird, wodurch eine weitere Kontaminationsquelle geschaffen ist.

Hier will die Erfindung Abhilfe schaffen. Der Erfindung liegt die Aufgabe zu Grunde, eine Vorrichtung zur bedarfsgerechten Desinfektion einer Armatur zum Öffnen einer Tür zu schaffen, welche eine selbsttätige Desinfektion bei fortwährender Bedienbarkeit der Tür ermöglicht, ohne dass ein personeller Aufwand erforderlich ist. Gemäß der Erfindung wird diese Aufgabe durch die Merkmale des Patentanspruchs 1 gelöst.

Mit der Erfindung ist eine Vorrichtung zur bedarfsgerechten Desinfektion einer Armatur zum Öffnen einer Tür geschaffen, welche bei fortwährender Bedienbarkeit der Tür selbsttätig desinfizierend ist, ohne dass ein personeller Aufwand erforderlich ist. Durch das Vorsehen einer UV-Strahlungsquelle zur Bestrahlung der Armatur ist eine wirksame Desinfektion bewirkt. Der Vorteil einer solchen UV-Desinfektion ist, dass gegen UV-Strahlung keine Resistenzen entwickelt werden können. Dadurch können auch chlorresistente Krankheitserreger, wie Cryptosporidien mit UV-Strahlung inaktiviert werden. Die UV-Strahlung wird von der DNA der Erreger absorbiert, zerstört deren Struktur und inaktiviert lebende Zellen. Mikroorganismen, wie Viren, Bakterien, Hefen und Pilze werden mit UV-Strahlung in Sekunden abgetötet. Durch die Abschirmvorrichtung, welche auch eine Nachrüstung bestehender Objekte, wie Türarmaturen ermöglicht, ist eine temporäre Bestrahlung der Betätigungsfläche ermöglicht, ohne dass UV-Strahlung in den Sichtbereich von in der Nähe befindlichen Personen gelangen kann. Hierdurch ist den gesetzlichen Bestimmungen Rechnung getragen.

Bevorzugt ist das UV-durchlässige Material Quarzglas. Dieses Material ist hinreichend stabil, ermöglicht ein ansprechendes Design und weist darüber hinaus einen hohen Durchdringungsgrad von UV-Strahlung auf.

Bevorzugt ist die Abschirmung mit einem Antrieb verbunden, über den sie bewegbar ist. Hierdurch ist eine selbsttätige Überdeckung der mit UV-Strahlung zu beaufschlagenden Fläche ermöglicht. Nach Abschluss des Desinfektionsvorgangs ist eine selbsttätige Freigabe der Betätigungsfläche durch automatische Entfernung der Abschirmvorrichtung ermöglicht.

In weiterer Ausgestaltung der Erfindung ist der Antrieb mit einem Sensor zur Detektierung einer Berührung der Betätigungsfläche verbunden. Hierdurch ist eine Steuerung des Antriebs in Abhängigkeit der Frequenz der Betätigungen ermöglicht. Somit kann nach jedem Kontakt einer Person mit der Betätigungsfläche automatisch der Desinfektionsvorgang gestartet werden,

Vorteilhaft emittiert die UV-Strahlungsquelle eine Strahlung im Wellenlängenbereich von 200 nm bis 300 nm, bevorzugt 240 nm bis 280 nm. In diesen Wellenlängenbereichen ist die UV-Desinfektion besonders wirksam.

Besonders vorteilhaft imitiert die UV-Strahlungsquelle eine Strahlung mit einer Wellenlänge von 254 nm. Es wurde gefunden, dass bei dieser Wellenlänge der Absorbtionsgrad der DNA am Größten ist, wodurch eine besondere Effizienz des Desinfektionsvorgangs bewirkt ist.

Andere Weiterbildungen und Ausgestaltungen der Erfindung sind in den übrigen Unteransprüchen angegeben. Ein Ausführungsbeispiel der Erfindung ist in der Zeichnung dargestellt und wird nachfolgend im Einzelnen beschrieben. Es zeigen:

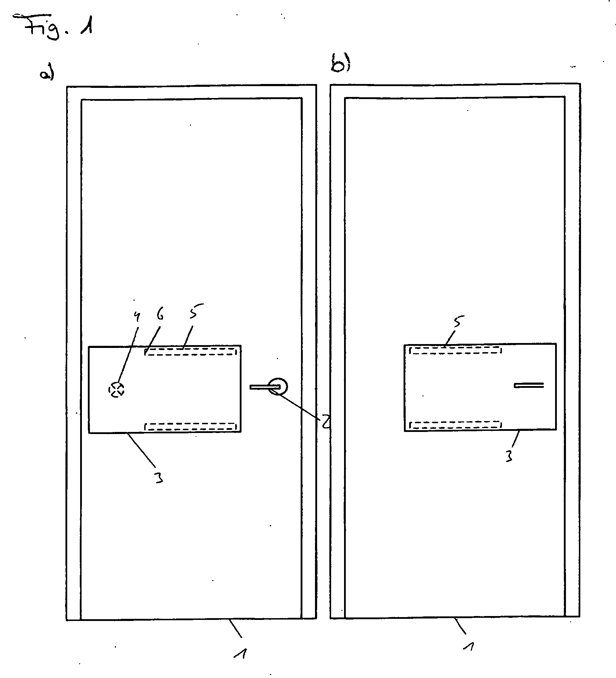

- Fig. 1: Die Darstellung einer Tür mit selbstdesinfizierender Türarmatur nicht gemäß der Erfindung a) in Bereitschaftsstellung b) im Desinfektionsbetrieb;
- Fig. 2: die Darstellung einer Tür mit zwei selbstdesinfizierenden rotierbaren Türarmaturen mit Abschirmung;
- Fig. 3: die Darstellung einer Tür mit zwei selbstdesinfizierenden ver- schiebbaren Türarmaturen mit Abschirmung;
- Fig. 4: die Darstellung eines selbstdesinfizierenden Betätigungsfeldes und
- Fig. 5: die Darstellung einer selbstdesinfizierenden Betätigungseinheit mit zwei Betätigungsfeldern und einer Abschirmung zur wechselseitigen Desinfektion.

Die als Ausführungsbeispiel gewählte Türarmatur besteht im Wesentlichen aus einem Türgriff 2 und einer Abschirmvorrichtung 3, in die eine UV-Strahlungsquelle 4 integriert ist und die über den Griff 2 bewegbar ist. Die so gebildete Armatur ist an einer Tür 1 montiert.

Im Ausführungsbeispiel ist die Strahlungsquelle 4 innerhalb der Abschirmvorrichtung 3 angeordnet und wird mit dieser nach Betätigung des Türgriffs 2 über den Türgriff 2 bewegt. Eine derart ausgebildete Abschirmvorrichtung kann auch zur Nachrüstung bereits vorhandener Türgriffe eingesetzt werden. Im Ausführungsbeispiel emittiert die UV-Strahlungsquelle eine Strahlung mit einer Wellenlänge von 254 nm.

Die Abschirmvorrichtung 3 ist im Ausführungsbeispiel aus Edelstahl hergestellt und ist im Wesentlichen als quaderförmige Hülse ausgebildet, Die Abschirmvorrichtung 3 ist auf einer Schiene 5 über einen - nicht dargestellten - Antrieb über den Türgriff 2 verfahrbar, so dass dieser vollständig gegenüber der Außenumgebung abgeschirmt ist. Der - nicht dargestellte - Antrieb ist mit einem - nicht dargestellten - Sensor verbunden, welcher in dem Griff 2 integriert ist und der geeignet ist, eine Berührung des Griffs 2 zu detektieren. Der- nicht dargestellte - Antrieb der Abschirmvorrichtung 3 ist weiterhin mit einem Timer versehen, wodurch ein Zeitversatz zwischen Berührung des Türgriffs 2 und der Bewegung der Abschirmvorrichtung 3 über den Türgriff 4 einstellbar ist.

Ist die Abschirmvorrichtung 3 vollständig über den Griff 2 bewegt, so wird über einen am Ende der Führungsschiene 5 befindlichen Anschlagtaster 6 die Strahlungsquelle 4 aktiviert, wodurch der Desinfektionsvorgang initiiert wird. Alternativ kann auch ein Schrittmotor vorgesehen sein, der nach einer vorgegebenen Bewegungssequenz ohne das Erfordernis eines Anschlagtasters 6 stoppt. Nach Ablauf einer in dem vorgenannten Timer definierbaren Zeitspanne wird die Abschirmvorrichtung wieder vom Türgriff 2 wegbewegt, wodurch über den Taster 6 die Strahlungsquelle 3 deaktiviert wird. Der so desinfizierte Griff 2 ist somit für die nächste Betätigung freigegeben.

In einer anderen Ausgestaltung ist der als Türöffner verwendete Türgriff 2 aus Quarzglas hergestellt. Er umschließt eine UV-Strahlungsquelle 4, welche derart ausgebildet ist, dass sie eine nahezu vollständige Bestrahlung der umlaufenden Betätigungsfläche des Türgriffs 2 ermöglicht.

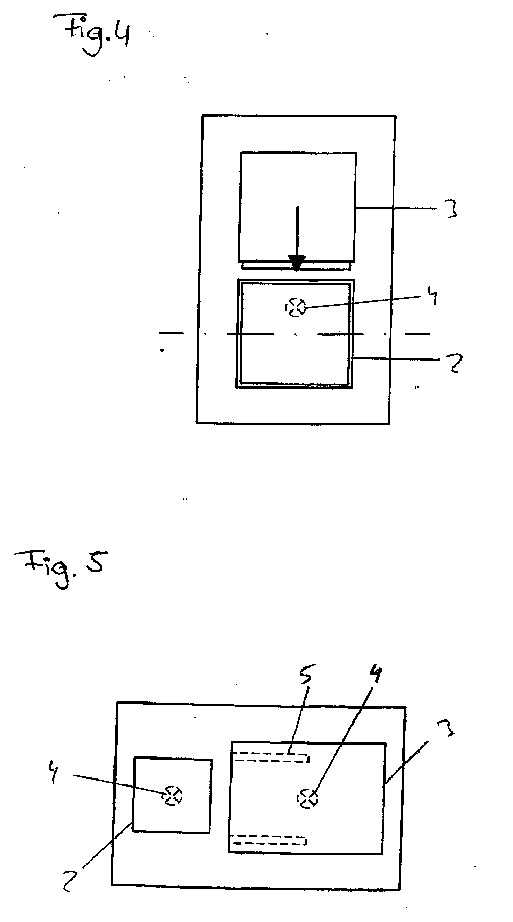

In einer weiteren Ausbildung kann der Türöffner auch als Bedienfeld ausgebildet sein (vgl. Fig. 4 und 5). In gleicher Weise kann eine Abschirmvorrichtung vorgesehen sein, welche über einen Antrieb über die Sensorplatte bewegbar ist, so dass die von einer Strahlungsquelle ausgesendete UV-Strahlung von der Außenumgebung abgeschirmt ist. Dabei kann die Strahlungsquelle sowohl innerhalb der Abschirmvorrichtung als auch hinter der Tasterplatte angeordnet sein. Bei Anordnung der UV-Strahlungsquelle hinter der Tasterplatte ist die Tasterplatte aus Quarzglas hergestellt, so dass eine Diffusion der elektromagnetischen UV-Strahlung ermöglicht ist.

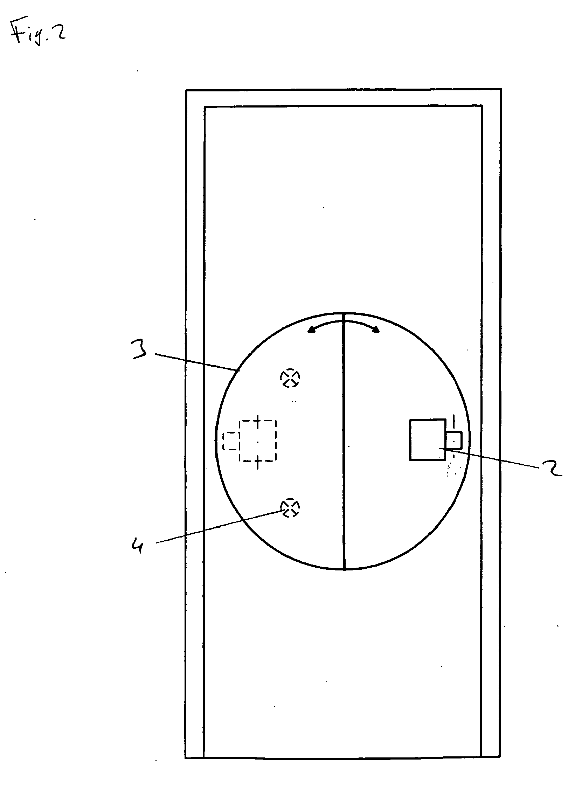

Im Ausführungsbeispiel gemäß Fig. 2 sind an der Tür 1 zwei Griffe 2 angebracht, wobei eine halbkreisförmige Abschirmung 3 angeordnet ist, welche mit einer UV-Strahlungsquelle 4 versehen ist. Die Griffe 2 sind auf einer Kreisscheibe um deren Kreismittelpunkt rotierbar gelagert, so dass jeweils ein Griff unter die Abschirmung 3 zur Desinfektion bewegbar ist, wobei gleichzeitig der andere der beiden Griffe 2 die ursprüngliche Position des ersten Griffs zur Bedienung einnimmt.

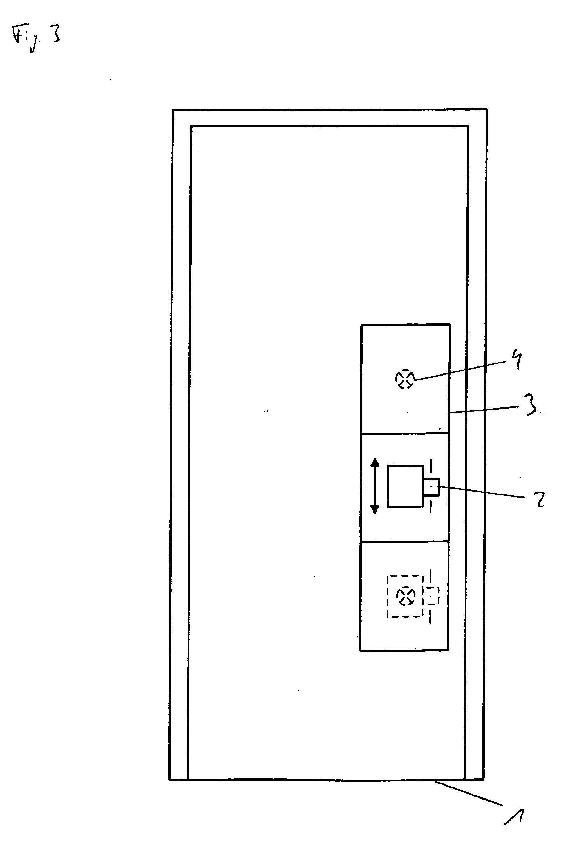

Im Ausführungsbeispiel gemäß Fig. 3 sind die beiden Griffe 2 vertikal verschiebbar angeordnet und unter eine von zwei beidseitig einer mittleren Schließposition angeordneten Abschirmungen zur Desinfektion verfahrbar, wobei der jeweils andere der beiden Griffe 2 zur Bedienung in die mittlere Schließposition bewegbar ist.

## Patentansprüche

1. Vorrichtung zur bedarfsgerechten Desinfektion eines Objekts, umfassend eine Abschirmung (3), die relativ zum Objekt bewegbar angeordnet ist, sodass das Objekt von der Abschirmung (3) umschließbar ist, wobei in der Abschirmung eine UV-Strahiungsquelle (4) angeordnet ist, mittels der das Objekt nach außen abgeschirmt mit UV-Strahlung beaufschlagbar ist, wobei das Objekt eine Armatur zum Öffnen einer Tür (1) ist, deren Betätigungsfläche (2) von der Abschirmung (3) während der Bestrahlung mit UV-Strahlung nach außen abschirmbar ist, **dadurch gekennzeichnet, dass** an der Tür (1) zwei Armaturen derart verschiebbar oder auf einer Kreisscheibe um deren Kreismittelpunkt rotierbar angeordnet sind, dass jeweils eine Armatur unter die Abschirmung (3) bewegbar ist, wobei die andere der beiden Armaturen die ursprüngliche Position der ersten Armatur einnimmt.

2. Vorrichtung nach Anspruch 1, **dadurch gekennzeichnet, dass** die UV-Strahlungsquelle (4) eine Strahlung im Wellenlängenbereich von 200 nm bis 300 nm, bevorzugt 240 nm bis 280 nm emittiert.

3. Vorrichtung nach Anspruch 2, **dadurch gekennzeichnet, dass** die UV-Strahlungsquelle (4) eine Strahlung mit einer Wellenlänge von 254 nm emittiert.

## Claims

1. Device for disinfection of an object as required, which device comprises a shielding means (3) which is disposed so that it can be moved relative to the object to enable the object to be enclosed by the shielding means (3), in which shielding means is disposed an UV radiation source (4) by means of which the object, which is shielded against the outside, can be irradiated by UV radiation, where the object is a means for opening a door (1) the actuating surface (2) of which opening means can during irradiation with UV radiation be shielded against the outside by the shielding means (3), **characterised in that** on the door (1) two opening means are so displaceably disposed or can be so rotated about the centre point of a circular plate that either of the two opening means can be moved under the shield (3), while the other of the two opening means takes up the original position of the first opening means.

2. Device in accordance with claim 1, **characterised in that** the UV radiation source (4) emits radiation in the wavelength range 200 nm to 300 and preferably 240 nm to 280 nm.

3. Device in accordance with claim 2, **characterised in that** the UV radiation source (4) emits radiation with a wavelength of 254 nm.

## Revendications

1. Dispositif de désinfection d'un objet par rayonnement ultraviolet, adaptée aux besoins, comprenant un blindage (3) agencé déplaçable relativement à l'objet, de sorte que l'objet soit entourable par le blindage (3), sachant qu'est agencée dans le blindage une source (4) de rayonnement ultraviolet, ledit blindage permettant d'exposer l'objet aux rayons ultraviolets sans que ces derniers ne puissent gagner l'extérieur, l'objet étant une robinetterie servant à ouvrir une porte (1), dont la surface d'actionnement (2) peut être rendue blindée par rapport à l'extérieur par le blindage (3) pendant l'exposition au rayonnement ultraviolet, **caractérisé en ce que** deux robinetteries contre la porte (1) sont agencées déplaçables ou rotatives autour du centre d'un disque rotatif de sorte qu'une robinetterie peut être chaque fois déplacée sous le blindage (3), sachant que l'autre des deux robinetteries vient occuper la position originelle de la première robinetterie.

2. Dispositif selon la revendication 1, **caractérisé en ce que** la source (4) de rayonnement ultraviolet est un rayonnement dont la longueur d'onde se situe dans la plage des 200 à 300 nanomètres, de préférence entre 240 et 280 nanomètres.

3. Dispositif selon la revendication 2, **caractérisé en ce que** la source (4) de rayonnement ultraviolet émet un rayonnement dont la longueur d'onde est de 254 nanomètres.
